Europäisches Patentamt

European Patent Office

Office européen des brevets

(11) Veröffentlichungsnummer: **0 230 043**
A2

# (12) EUROPÄISCHE PATENTANMELDUNG

(21) Anmeldenummer: 86117926.5

(22) Anmeldetag: 23.12.86

(51) Int. Cl.⁴: **C 12 P 7/42**
// (C12P7/42, C12R1:02)

(30) Priorität: 10.01.86 DE 3600476

(43) Veröffentlichungstag der Anmeldung: 29.07.87
Patentblatt 87/31

(84) Benannte Vertragsstaaten: AT BE CH DE FR GB IT LI NL
SE

(71) Anmelder: Kernforschungsanlage Jülich Gesellschaft
mit beschränkter Haftung, Postfach 1913,
D-5170 Jülich 1 (DE)
Anmelder: Henkel Kommanditgesellschaft auf Aktien,
Postfach 1100 Henkelstrasse 67,
D-4000 Düsseldorf-Holthausen (DE)

(72) Erfinder: Giesel-Bühler, Hermine, Dr. Dipl.-Biol.,
Max-Born-Strasse 43, D-4000 Düsseldorf 13 (DE)
Erfinder: Bartsch, Frank Olaf, Dipl.-Biol.,
Helfmannstrasse 60, D-6100 Darmstadt (DE)
Erfinder: Kneifel, Helmut, Prof. Dr. Dipl.-Chem.,
Franziskusstrasse 5, D-5170 Jülich (DE)
Erfinder: Sahm, Hermann, Prof. Dr. Dipl.-Biol.,
Wendelinusstrasse 71, D-5170 Jülich (DE)
Erfinder: Schmid, Rolf, Dr. Dipl.-Chem., Am
Nettchesfeld 30, D-4000 Düsseldof 13 (DE)

(54) **Verfahren zur Hydratisierung ungesättigter Carbonsäuren.**

(57)   Die Addition von Wasser an eine $C_9C_{10}$-Doppelbindung
von ein- oder mehrfach ungesättigten längerkettigen Carbonsäuren gelingt biotechnologisch mit Hilfe eines Mikroorganismus der Species Acetobacterium woodii. Besonders
geeignet sind Acetobacterium woodii ATCC 29683 sowie
dessen Mutanten oder Varianten gleicher Wirkungsrichtung. Dieses Verfahren eignet sich insb. zur Umsetzung
mehrfach ungesättigter Carbonsäuren mit 12–24 Kohlenstoffatomen, insb. von Linolsäure und/oder Linolensäure.

EP 0 230 043 A2

ACTORUM AG

## Verfahren zur Hydratisierung ungesättigter Carbonsäuren

Die Erfindung betrifft ein Verfahren zur Umsetzung von ein- oder mehrfach ungesättigten längerkettigen Carbonsäuren mit einer $C_9C_{10}$-Doppelbindung unter Addition von Wasser an diese $C_9C_{10}$-Doppelbindung, das insb. die Herstellung ungesättigter Hydroxycarbonsäuren erlaubt.

Ungesättigte Hydroxycarbonsäuren sind bekannt. Ein wichtiger Vertreter dieser Substanzklasse ist die Rizinolsäure (12-Hydroxy-octadecensäure). Rizinolsäure hat eine Vielzahl technischer Anwendungen und wird dafür fast ausschließlich aus Rizinusöl hergestellt. Rizinusöl steht jedoch nicht in unbegrenzter Menge zur Verfügung, so daß ein Bedarf an vergleichbaren Austauschprodukten auf anderer natürlicher oder synthetischer Basis besteht.

Weiterhin sind eine Reihe chemischer Verfahren bekannt, die es erlauben, mehrfach ungesättigte Carbonsäuren in Hydroxycarbonsäuren zu überführen. Aufgrund der Reaktionsträgheit der CC-Doppelbindung müssen dabei jedoch entweder drastische Bedingungen oder sehr aktive Agentien eingesetzt werden. Dadurch ist es sehr schwierig, nur an eine Doppelbindung (formal) Wasser anzulagern. Die im Labormaßstab durchführbaren Verfahren,

1.800 G

nö/ha

2

wie Hydroborierung, Epoxidation und anschließende Reduktion oder Wasseranlagerung in Gegenwart starker Säuren konnten sich daher technisch nicht durchsetzen.

Es besteht daher Bedarf nach einem Verfahren, das es erlaubt, ungesättigte Alkancarbonsäuren und/oder deren Derivate, insb. die gut zugänglichen Vertreter wie Linolsäure oder Linolensäure unter physiologischen Bedingungen in entsprechende ungesättigte Hydroxycarbonsäuren oder deren Derivate überführen zu können.

Aufgabe der Erfindung ist es, ein derartiges Verfahren bereitzustellen, insb. ist es Aufgabe der Erfindung, ein Verfahren zur Umwandlung von mehrfach ungesättigten Alkancarbonsäuren mit 12 - 24 C-Atomen und/oder deren Derivaten bereitzustellen.

Gegenstand der Erfindung ist somit ein Verfahren zur Umsetzung von ein- oder mehrfach ungesättigten längerkettigen Carbonsäuren mit einer $C_9C_{10}$-Doppelbindung unter Addition von Wasser an diese $C_9C_{10}$-Doppelbindung, dadurch gekennzeichnet, daß die Anlagerung mit Hilfe eines Mikroorganismus der Species Acetobacterium woodii erfolgt.

Bei dem im erfindungsgemäßen Verfahren eingesetzten Acetobacterium woodii handelt es sich um ein strikt anaerobes, gram-(+)Doppelstäbchen, welches chemolithoautotroph in Gegenwart von $H_2$ und $CO_2$ wachsen kann. Der Organismus ist darüber hinaus auch zur Umsetzung von Zuckern als Kohlenstoffquelle befähigt.

Für das erfindungsgemäße Verfahren besonders geeignet ist ein Stamm <u>Acetobacterium woodii</u>, ATCC 29683 entsprechend DSM 1030.

Aus diesem Stamm lassen sich durch die dem auf diesem Arbeitsgebiet tätigen Fachmann bekannten Mutations- und Selektionsverfahren Mutanten mit gewünschtenfalls größerer Produktivität gewinnen. Zur Mutation werden die für Anaerobier anwendbaren Verfahren eingesetzt. So kann man beispielsweise Kulturen des Mikroorganismus harter Strahlung oder chemischen Stoffen mit mutagener Wirkung aussetzen, die so behandelten Kulturen verdünnen, auf geeigneten Nährböden anzüchten. Unter den erhaltenen Mutanten kann selektiert werden. Oder man kann beispielsweise mit bekannten gentechnischen Methoden die Copyzahl des Gens oder die Effektivität des Promotersystems erhöhen, um eine höhere Produktivität zu erhalten.

Im erfindungsgemäßen Verfahren können eine Vielzahl von ein- und mehrfach ungesättigten Carbonsäuren mit einer CC-Doppelbindung in 9 - 10 Stellung und deren Derivate wie Ester, Salze oder auch Amide etc. eingesetzt werden. Bevorzugt werden jedoch Carbonsäuren mit mehr als einer CC-Doppelbindung und 12 - 24 Kohlenstoffatomen eingesetzt. Unter diesen wiederum sind unverzweigte Verbindungen bevorzugt. So sind ausgezeichnete Rohstoffe mehrfach ungesättigte Fettsäuren wie beispielsweise Linolsäure und Linolensäure. Geeignet sind aber auch Carbon-säuren mit kurzen ($C_1$ bis $C_3$) Verzweigungen.

4

Zur Durchführung des Verfahrens züchtet man einen Stamm von Acetobacterium woodii und/oder dessen Mutanten und/oder Varianten, die zur Anlagerung von Wasser an $C_9C_{10}$-Doppelbindungen ungesättigter Carbonsäuren mit einer oder mehreren Doppelbindungen befähigt sind, unter anaeroben Bedingungen an. Bei der Anzucht kann als C-Quelle $CO_2$ in Verbindung mit $H_2$ dienen. Es kann jedoch auch auf mehrfunktionellen Alkoholen, insb. Glycerin, Hexosen, wie Glucose angezüchtet werden. Nach einem bevorzugten Verfahren werden Zellen der frühen stationären Phase unter anaeroben Bedingungen geerntet, 1mal mit anaerobisiertem Puffer gewaschen und in Puffer unter Schutzgas ($N_2$, Ar) mit den mehrfach ungesättigten Fettsäuren mit oder ohne Zusatz von einer Kohlenstoff- und Energiequelle inkubiert.

Nach einer besonders bevorzugten Ausführungsform der Erfindung werden die ungesättigten Carbonsäuren in Gegenwart eines Emulgiermittels zur Zellsuspension, die auch eine Kohlenstoff- und Energiequelle und gewünschtenfalls übliche Zusatz- und Hilfsstoffe enthält, gegeben. Bevorzugt sind hier physiologisch verträgliche Emulgiermittel, und zwar insb. nichtionische Emulgatoren. Geeignete Emulgatoren sind beispielsweise Rinderserumalbumin, Sorbitanfettsäureester, wie Sorbitanmonooleat, Ethoxylierungsprodukte von Sorbitanfettsäureester, wie beispielsweise das Handelsprodukt Tween[R] 80 sowie Alkylglycoside mit 8 - 22, vorzugsweise 8 - 14 C-Atomen im Alkylrest. Weitere nichtionische Emulgatoren sind ethoxylierte langkettige Alkohole, z.B. ethoxylierte

5

Fettalkohole mit 12 - 18 Kohlenstoffatomen und 4 - 100 Ethylenglycoleinheiten in der Polyethylenglycoletherkette oder auch ethoxylierte entsprechende Alkylphenole. Darüberhinaus können anionische Emulgatoren, insb. solche mit Sulfo- oder Sulfonsäuregruppen eingesetzt werden.

Zur Isolierung der Produkte werden die Zellen abgetrennt und der Überstand mit einer nichtwassermischbaren Flüssigkeit extrahiert. Auch andere Aufarbeitungsmöglichkeiten, beispielsweise die Abtrennung der Hydroxycarbonsäuren über eine kationische Ionenaustauschersäule oder durch Adsorption an Feststoffe wie Zeolithe, sind denkbar.

Nach einer weiteren Ausführungsform der Erfindung werden die Mikroorganismen zunächst in einem Nährmedium angezüchtet und dann in üblicher Weise mit Hilfe von organischen oder anorganischen Trägern immobilisiert. Die Umsetzung der mehrfach ungesättigten Carbonsäuren und/oder deren Derivate erfolgt dann in analoger Weise an den immobilisierten, aber lebensfähigen Zellen, die dann z.B. an einem gekörnten Träger fixiert in einem von Reaktanten und Hilfsstoffen durchströmten Reaktionsrohr angeordnet werden.

Beispiel

Acetobacterium woodii ATCC 29 683 entsprechend DSM 1030 wurde unter anaeroben Bedingungen in einem Nährmedium der folgenden Zusammensetzung angezüchtet:

6

| | | |
|---|---|---|
| Glucose oder | 10 | g |
| Glycerin | 6 | g |
| $NH_4Cl$ | 1 | 9 |
| Hefeextrakt (Difco) | 2 | g |
| $MgSO_4 . 7 H_2O$ | 0,1 | g |
| Wolfe's Mineral Solution | 20 | ml |
| Resazurin 0,1 % | 1 | ml |
| $H_2O$ bidest ad | 1000 | ml |
| $N_2/CO_2$-Atmosphäre | | |
| pH 7,4 | | |

Die Glucose wurde in einem kleinen Volumen $H_2O$ gelöst und getrennt autoklaviert.

| | | |
|---|---|---|
| $NaHCO_3$-Lösung 100 g/l | 100 | ml |
| $Na_2S . 7 H_2O$-Lösung 25 g/l | 10 | ml |

Diese Lösungen wurden getrennt autoklaviert und vor dem Beimpfen zugesetzt.

Zellen der frühen stationären Phase (Wachstumsdauer ca. 40 h) wurden entweder sofort weiterverwendet oder bis zur Weiterverwendung bei -20°C unter Argon gelagert.

Herstellung der Fettsäure-Lösungen:

1 ml Kaliumphosphatpuffer (KPP) 0,1 M pH 7,0 wurde durch Einleiten von Argon für 20 min anaerobisiert und 120 mg Rinderserumalbumin (RSA) als Detergens hinzugefügt.

Nach Lösen des RSA wurde unter Begasung die entsprechende Fettsäure zugegeben. Die Konzentrationen
dieser Substrate in der RSA-Lösung betrug jeweils
100 mM. Als Alternative zum RSA kann auch Tween 80
(3%ige Lösung) als Detergens verwendet werden.

Die Warburg-Gefäße enthielten:

Reaktionsraum     3    ml Bakteriensuspension (0,3 g
                       Bakterienfeuchtmasse +
                  2,7  ml 0,1 M KPP pH 7,0)
                  0,05 ml Tetracyclin (1,5 mg/ml)

Seitenarm         0,3  ml Fettsäure
(Substrat)             (100 mM in RSA)

Der Ansatz wurde durch Zukippen des Substrates
gestartet. Nach 15 - 18 Stunden wurde der Gefäßinhalt zentrifugiert und der Überstand bei
-20°C bis zur Analyse aufbewahrt.

Zur Herstellung größerer Quantitäten der Biotransformationsansätze wurden Warburg-Gefäße
mit 90 ml Fassungsvermögen verwendet. Die Konzentrationen aller Substanzen im Biotransformationsansatz sowie die Inkubationsdauer blieben
unverändert.

Zur Aufreinigung der Biotransformationsansätze
mit HPLC wurde ein spezielles Extraktionsverfahren
notwendig:

10 ml der Ansätze wurden mit 10 ml $H_2O$ bidest
verdünnt und 20 ml Essigsäureethylester extrahiert.

8

Die Essigesterphase wurde aufgefangen und die wäßrige Phase nochmals mit 10 ml Essigester extrahiert. Die Essigesterphase wurde gesammelt und eingeengt, die Fettsäuren mit 10 % Acetylchlorid/Methanol 90 % methyliert und über eine Kieselgel-Säule vorgereinigt. Elutionsmittel war Essigester.

Die Aufreinigung erfolgte mittels HPLC:

| Pumpe | : Waters M 6000 A |
| Injektor | : U 6 K |
| Säule | : Knauer RP 18   30 . 1,6 cm |
| Detektion | : Uvicord S 11 - Photometer LKB |
| Wellenlänge | : 206 nm |
| Integrator | : Shimadzu CK 3 A |
| Laufmittel | : Methanol |
| Temperatur | : 20°C |
| Fluß | : 6 ml/min |
| Druck | : 40 atm. |

In der vorstehenden Weise wurde aus Linolsäure 10-Hydroxy-12-octadecensäure (Nachweis durch NMR) hergestellt.

In analoger Weise wurde Linolensäure umgesetzt.

9

Patentansprüche

1.  Verfahren zur Umsetzung von ein- oder mehrfach ungesättigten längerkettigen Carbonsäuren mit einer $C_9C_{10}$-Doppelbindung unter Addition von Wasser an diese $C_9C_{10}$-Doppelbindung, dadurch gekennzeichnet, daß die Anlagerung mit Hilfe eines Mikroorganismus der Species Acetobacterium woodii erfolgt.

2.  Verfahren nach Anspruch 1, dadurch gekennzeichnet, daß Acetobacterium woodii ATCC 29683 entsprechend DSM 1030 und/oder dessen zur Anlagerung von Wasser an $C_9C_{10}$-Doppelbindungen ungesättigter Carbonsäuren befähigte Mutanten oder Varianten eingesetzt werden.

3.  Verfahren nach den Ansprüchen 1 und 2, dadurch gekennzeichnet, daß Carbonsäuren mit mehr als einer CC-Doppelbindung und 12 - 24 C-Atome eingesetzt werden.

4.  Verfahren nach den Ansprüchen 1 - 3, dadurch gekennzeichnet, daß unverzweigte Carbonsäuren mit mehr als einer CC-Doppelbindung eingesetzt werden, die eine gerade Anzahl von C-Atomen aufweisen, insb. Linolsäure und/oder Linolensäure.